# EUROPEAN PATENT SPECIFICATION

(11) **EP 0 606 099 B1**
(45) Date of publication and mention of the grant of the patent: **15.09.1999**
(21) Application number: 94101041.5
(22) Date of filing: 30.07.1990
(51) Int. Cl.: F04B 43/08

(54) **Peristaltic pump**
Peristaltische Pumpe
Pompe peristaltique

(30) Priority: 31.07.1989 JP 19651589; 31.07.1989 JP 19651689
(43) Date of publication of application: 13.07.1994
(62) Divisional of application: 90114604.3
(73) Proprietor: TERUMO KABUSHIKI KAISHA, Tokyo (JP)
(72) Inventor: Okada, Shigeru, Fujinomiya-shi, Shizuoka (JP)
(74) Representative: Casalonga, Axel

(56) References cited:
- EP-A- 0 239 769
- EP-A- 0 283 614
- US-A- 4 867 744

## Description

The present invention relates to a peristaltic pump suitable for use in, for example, transfusion.

Hitherto, various peristaltic pumps have been proposed as disclosed, for example, in Japanese Unexamined Patent Publication No. 56-113084. In general, a known peristaltic pump has a plurality of cams, a plurality of pressing fingers actuated by the cams so as to move between a retracted position and an operative position, and a platen opposing the pressing fingers so as to carry a tube therebetween. In operation, the pressing fingers are successively actuated to the operative position by the cams so as to collapse the tube, whereby a liquid in the tube is displaced and transported, while the elastic tube is allowed to restore when relieved from the pressing force so as to expand the liquid passage, thus performing a pumping action.

In the peristaltic pump of the type described, problems are caused such as damaging of the tube due to excessive pressing, unduly large load posed on the cam actuating device, and so forth, as a result of any dimensional errors caused during manufacture of the components such as the cams, pressing fingers and the platen, as well as insufficiency in the locating precision of these components. In order to obviate these problems, it has been proposed to provide an elastic support member on the back side of the platen opposite to the pressing fingers, so that any excessively large pressing force is absorbed by the elastic deformation of the supporting member thereby relieving and unburdening the platen.

When the platen is unburdened, the pressing force to be relieved acts only on a local point on the tube which is immediately under a certain pressing finger which has been set to the operative position. Therefore, during the unburdening, the platen does not always make a translational movement. Namely, the platen may be moved at an inclination with respect to the plane of the group of pressing fingers. In such a case, the support of the tube on the platen is rendered unstable so as to impair the precision in the control of the flow rate of the liquid during transfusion.

With respect to this circumstance, Japanese Unexamined Utility Model Publication No. 62-20182 discloses a peristaltic pump which is improved to eliminate the above-described problems concerning the unburdening of the platen. In this improved peristaltic pump, any excessive force which would unduly collapse the tube is relieved not by a displacement of the platen but by elastic deformation of an elastic buffering portion of each pressing finger made of an elastic material. Thus, any excessive force applied to a pressing finger, which would otherwise be exerted on the tube, is effectively absorbed by the pressing finger itself.

A problem encountered with the known peristaltic pump disclosed in Japanese Unexamined Patent Publication No. 56-113084 is that it is not designed to stably set the entire part of the tube at proper position between the group of pressing fingers and the platen. This poses the following problems ① and ②.
① The tube winds in the space between the pressing fingers and the platen, so that the length of the portion of the tube pressed by each pressing finger may differ from the design length.
② The portion of the tube to be pressed by each pressing finger may partly placed out of the area where the pressing force by the pressing finger is exerted.

These problems ① and ② make it impossible to press successive sections of the tube of a predetermined length, thus impairing the precision of the flow rate control.

EP-A-0 283 614 discloses a peristaltic pump whicn comprises a plurality of cams which are carried and driven by a cam shaft, and a plurality of fingers urged by the cams against the tube. A platen is supporting the tube opposite to the group of fingers. A casing is pivotally connected with a housing and urged towards the housing by a spring. The cam shaft remains in the same position with the respect to the casing. The peristaltic fingers can either be moved by a rotation of the cam shaft urging the cams against the peristaltic fingers or they can be moved relative to the housing in response to a movement of the casing with respect to the housing. Alternatively, the motion of the peristaltic fingers can be caused combinedly in both ways (cf. column 12, lines 1 to 15). The above mentioned spring is provided for urging the rotation of the casing towards the housing in such a manner that the peristaltic fingers are held in guide means.

EP-A-0 239 769 is directed to a peristaltic pump in which a tube is supported by a platen opposite to a group of members eccentrically mounted on a shaft for pressing and collapsing the tube. The centers of the members are equidistant from the axis of the shaft with a similar angular spacing between the centers. Retaining resp. holding means are provided for retaining the tube in a predetermined position. However, the tube is held outside the region of action of the pressing members.

Accordingly an object of the present invention is to provide a peristaltic pump which enables a tube to be stably set at a proper position between a group of pressing fingers and a platen.

As claimed, there is provided a peristaltic pump comprising a plurality of eccentric cams, a cam shaft carrying the cams and capable of driving the cams, a plurality of fingers adapted to be driven by the cams so as to press and collapse a tube. A platen is arranged to oppose the group of fingers and capable of holding the tube between itself and the fingers. Further a tube holder is disposed between the fingers and the tube and/or between the platen and the tube and a tube holding means is provided capable of holding the tube in a predetermined position. According to the invention the tube holding means is provided on the surface of the tube holder contactable with the tube.

By this arrangement the entire portion of the tube received in the tube holder is correctly set so that the tube can be collapsed in a very stable manner by the successive pressing fingers thus enhancing the precision in the control of the flow rate of the liquid flowing through the tube.

According to an embodiment of the present invention the tube holder includes a sheet-type elastic member having a recess-type tube holding means formed in the surface. thereof contactable with the tube.

Furthermore, the tube holder can preferably include at least a pair of tube clamping portions projecting into the recess.

According to a further embodiment of the present invention, the peristaltic pump further comprises clamping fingers capable of pressing the clamping portions so as to enable the clamping portions to clamp the tube.

The peristaltic pump advantageously further comprises: a housing accommodating the cams, a cam shaft and the fingers and supporting a tube holder in such a manner that the tube holder is located at a position where it opposes the tube to be mounted; and a door supported on the housing for movement between open and close positions and carrying the platen in such a manner that the platen opposes the tube.

According to the present invention, the surface of the tube holder to be contacted by the tube can be made of a silicon rubber having a large coefficient of friction.

According the invention, consecutive longitudinal sections of the elastic tube on the platen are successively collapsed as the successive fingers of the finger group are activated, namely, the position on the elastic tube where the collapsing force is exerted is progressively moved in the longitudinal direction of the tube, so that the liquid in the tube is displaced, whereas the portion of the tube relieved from the collapsing force allows to resume its original form by its elasticity so as to expand the liquid passage therein to suck a new portion of the liquid, whereby a pumping action is performed.

By means of the peristaltic pump according to the invention, the tube is properly placed without any winding, by means of tube holding means on the tube holder, in such a manner that successive tube sections of the designed length are placed in the pressing regions of the successive fingers, whereby the tube is correctly pressed by the successive pressing fingers at the successive sections so as to provide a high precision control of the flow rate.

As claimed, the tube held by the tube holding means in the pump can be clamped by a pair of clamp portions which are disposed at a predetermined longitudinal position of the recess-type holding means, so that the successive sections of the tube can be properly located within the regions of the successive pressing fingers.

Preferrably the clamping portions of the tube holder are pressed by the clamping fingers so that the locating precision of the tube is further enhanced.

According to a further embodiment of the present invention, the cams, the pressing fingers, the platen and the tube holder are accommodated in the housing through the door, whereby a compact pump can be realized. Furthermore, the tube can be set on the tube holder and, hence, can be mounted very easily in the right space between the group of fingers and the platen when the door is opened and closed.

According to the present invention, the tube holder may advantageously have a surface formed of a silicon rubber capable of stably holding the tube, while the surface of the tube holder to be contacted by the pressing fingers is formed of a fluororesin having a small coefficient of friction, thus minimizing the wear of the portions of the tube holder to be contacted by the pressing fingers.

In the following the invention will be further elucidated by the description of preferred embodiments of the invention in conjuction with the accompanying drawings. In the drawings:
Fig. 1 is a schematic illustration of the whole of a peristaltic pump;
Fig. 2 is a sectional view showing a critical portion of the peristaltic pump of Fig. 1;
Fig. 3 is a longitudinal sectional view showing the critical portion of the peristaltic pump of Fig. 1;
Fig. 4 is an exploded perspective view showing critical portions of the peristaltic pump of Fig. 1;
Fig. 5 is a schematic illustration of operation of pressing fingers of the peristaltic pump;
Fig. 6 is a schematic illustration showing a guide plate and clamping fingers in the peristaltic pump;
Fig. 7 is a schematic illustration of operation of pressing fingers;
Fig. 8 is a schematic illustration of a guide plate; and
Fig. 9 is a cross-sectional view of another embodiment of the peristaltic pump.

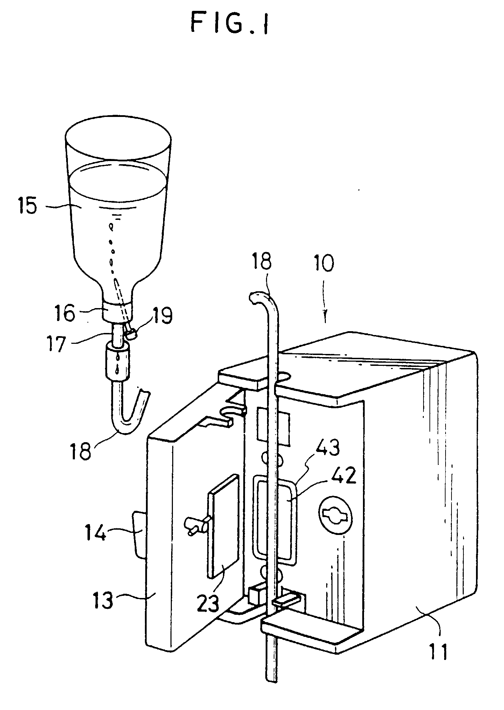

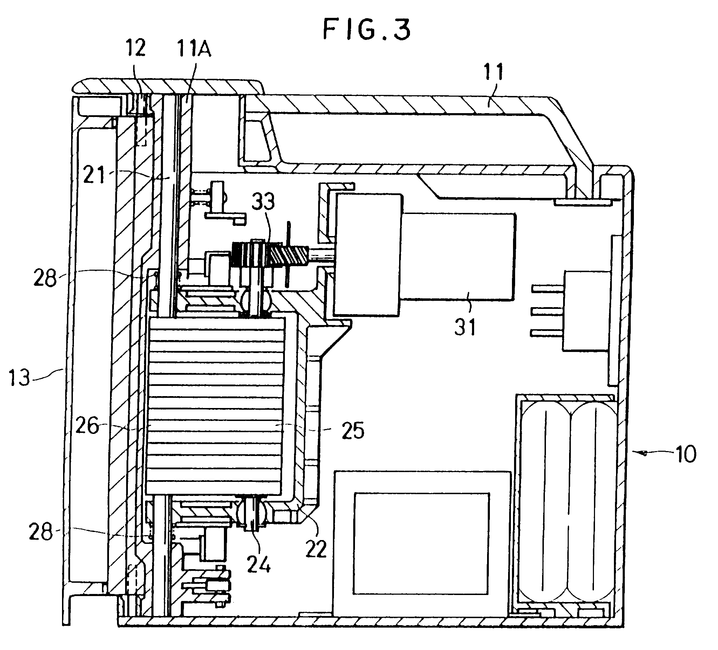

Referring to Figs. 1 to 3, a peristaltic pump 10 has a housing 11 and a door 13 connected to the housing 11 through a door shaft 12 for pivotal movement between an open position and a closed position. The door 13 is provided with a knob 14 which can be rotated to lock and unlock the door 13 in the closed position.

The pump 10 is capable of effecting a pumping action on an intermediate portion of a tube 18 which is connected to a liquid guide needle 17 piercing a stop cock 16 of a transfusion vessel 15 shown in Fig. 1, so as to transfuse a liquid from the transfusion vessel 15 into the body of a patient. Numeral 19 denotes a ventilation needle.

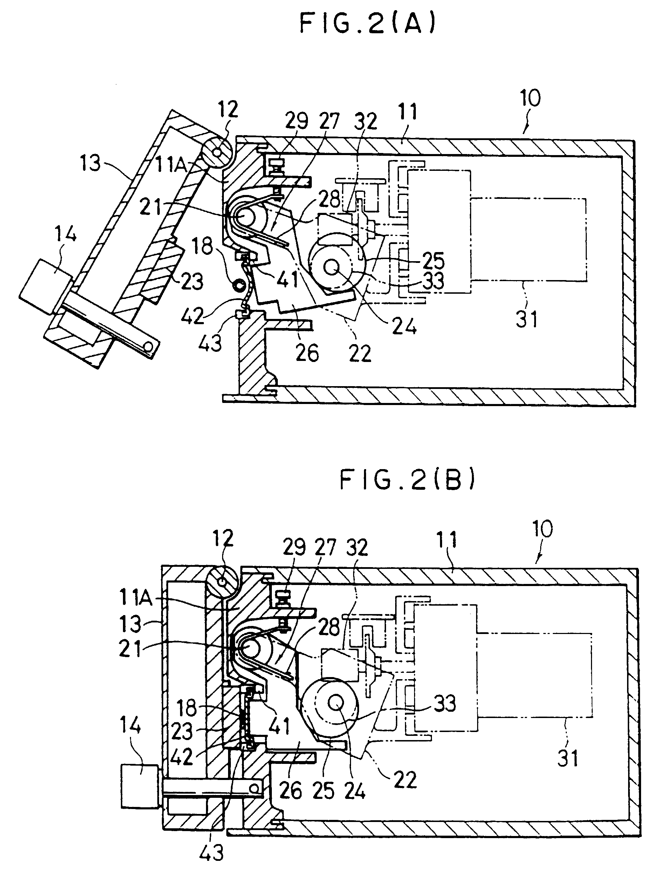

As shown in Figs. 2 to 4, the pump 10 has a movable member 22 rockably carried by a pivot shaft 21 which is held at its both ends by brackets 11A fixed to the housing 11. The pump 10 also has a platen 23 supported on the door 13.

The movable member 22 is rockably supported by the pivot shaft 21 for rocking motion toward or away from the platen 23, and has a cam shaft 24 which extends in parallel with the pivot shaft 21. The movable member 22 also incorporates a plurality of eccentric cams 25 fixedly mounted on the cam shaft 24 along the length thereof, and a plurality of fingers 26 pivotally suported about the pivot shaft 21 and adapted to be pressed by the eccentric cams 25.

In this state, each finger 26 is adapted to be actuated by a corresponding eccentric cam 25 between a retracted position and an operative position. When the door 13 is closed, the platen 23 can be located so as to oppose the group 26A of fingers 26 so as to carry the tube 18 which is disposed between the platen 23 and the fingers 26. The fingers are successively set to the operative position by the operation of the successive eccentric cams 25 so that the position where the tube 18 is collapsed by the fingers is progressively moved in the longitudinal direction of the tube. It is to be noted that the tube is normally closed by at least one finger 26. The arrangement is such that, when one of the fingers 26 has commenced its backward movement beyond the maximum collapse position, the next finger keeps the tube in the pressed condition so as to keep the internal liquid passage of the tube 18 closed until the above-mentioned finger 26 travels a distance large enough to open the portion of the internal liquid passage under this finger 26.

The pump 10 also has an urging means 27 which resiliently urges the movable member 22 toward the platen 23. The urging means can be composed of a torsion spring 28 (urging member) and an adjusting screw (adjusting member). In the illustrated embodiment, there are two torsion springs 28 which are arranged on the pivot shaft 21 at the respective sides of the movable member 22. Each torsion spring has one end engaging with the movable member 22, while the other end engages with the end of an adjusting screw 29 which is screwed to each bracket 11A fixed to the housing 11. In this state, the housing 11 carrying the brackets 11A to which the adjusting screws 29 are screwed and the platen 23 supported by the door 13 locked in the closed position on the housing 11 are immovable relative to each other. Consequently, the torsion springs 28 act between the movable member 22 and the platen 23. The adjusting screws 29 can be driven into and out of threaded holes formed in the brackets 11A so as to adjust the urging forces produced by the torsion springs 28.

The pump 10 also has a drive motor 31 fixed to the movable member 22 and having an output shaft carrying a worm gear 32 meshing with a worm wheel 33 which is fixed to one end of the cam shaft 24.

The arrangement is such that the cam shaft 24 is rotatingly driven by the drive motor 31 so as to rotate the eccentric cams 25 thereby activating successive fingers 26, whereby a pumping action is performed as explained before.

The peristaltic pump 10 of the present invention has a tube guide device as will be understood from the following description.

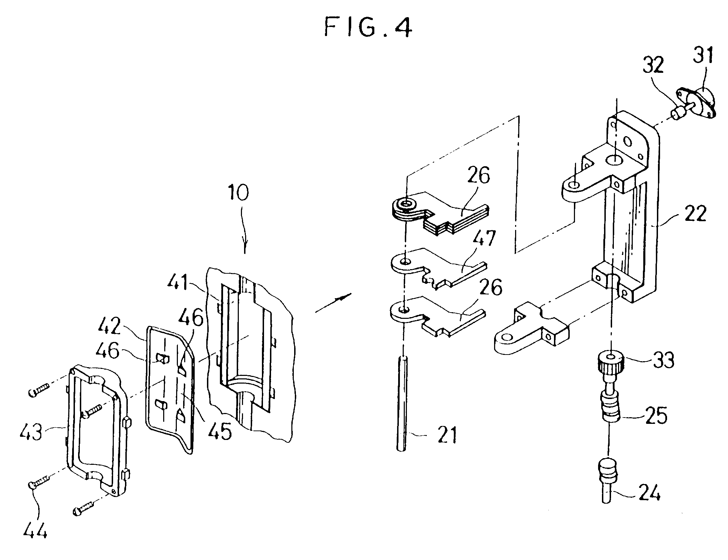

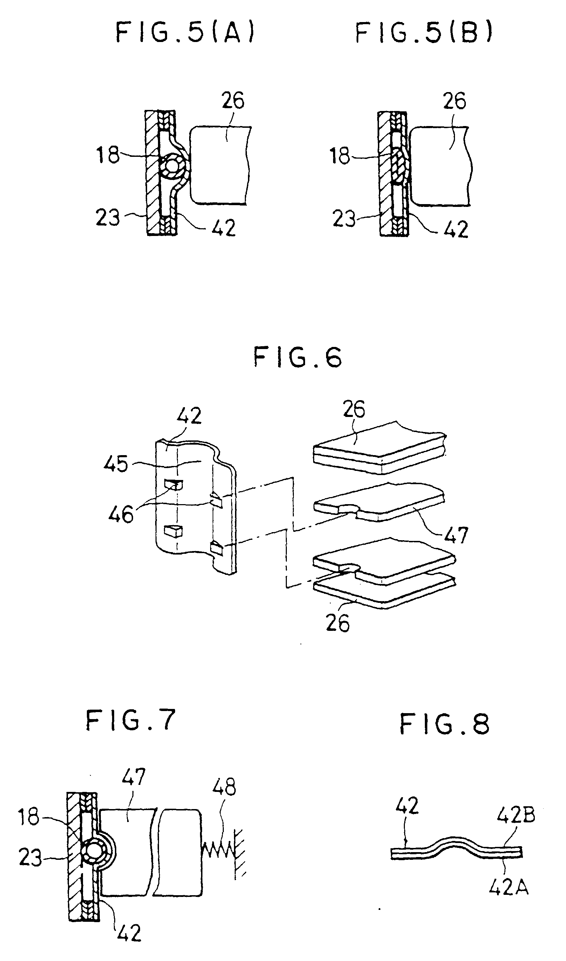

Namely, as shown in Fig. 4, each bracket 11A on the housing 11 has a pressing window 41 and a guide plate 42 for closing the pressing window 41. The guide plate 42 is fastened to the bracket 11A by means of screws 44 through a frame member 43 which presses the outer peripheral portion of the guide plate 42. As will be seen from Figs. 2A and 2B, the guide plate 42 is provided with a tube guide groove 45 formed in the surface thereof which faces the platen 23 when the door 13 is locked in the closed position on the housing 11 so as to set the tube 18. The guide plate 42 is made of a soft elastic material so as not to impede the collapsing action of the fingers 26 when the successive fingers are set to the operative position for collapsing the tube 18. Therefore, as shown in Fig. 5A, the tube 18 is received in and guided by the guide groove 45 of the guide plate 42 so as to be correctly set without winding, in such a manner that successive tube sections of a predetermined length are correctly located in the pressing regions of the successive fingers. Consequently, the tube is stably collapsed by the successive fingers as these fingers are set to the operative position, whereby a higher precision of control of the liquid flow rate can be obtained, as will be seen from Fig. 5B.

As shown in Fig. 4, the guide plate 42 is provided with two pairs of clamping portions 46 spaced in the longitudinal direction of the guide groove 45 and formed on opposing walls of the guide groove 45 so as to project into the guide groove 45 thereby to clamp the tube 18. The pair of clamping portions 46 of each pair oppose each other so as to define therebetween a gap substantially equal to the outside diameter of the tube 18. Thus, the clamping portions 46 serve to locate the tube 18 in the groove 45 more precisely within the region where the collapsing forces are exerted by the successive fingers 26.

As shown in Figs. 6 and 7, the peristaltic pump of the present invention has a plurality of clamping fingers 47 each of which are disposed at positions corresponding to the respective pairs of opposing clamping portions 46 on the guide plate 42 and between two adjacent pressing fingers 26. Each clamping finger 47 is configured to have a recess which surrounds the portion of the guide plate 42 forming the guide recess 45, so as not to collapse the tube 18 and so as to press the clamping portions 46. As schematically shown in Fig. 7, each clamping finger 47 is urged towards the clamping portions 46 by means of a back-up spring 48 acting between itself and the housing 11, thereby enhancing the locating effect of the clamping portions 46 for locating the tube 18.

The guide plate 42 is made of, for example, a rubber and the surface of the guide plate 42 opposite to the guide recess 45, i.e., the surface to be pressed by the fingers 26, is embossed so as to reduce the coefficient of friction, thereby suppressing friction due to contact with the fingers 26.

As shown in Fig. 8, the guide plate 42 may be a composite member the surface of which contactable with the tube 18 is formed from a material having a large coefficient of friction, e.g. a silicon rubber 42A, so as to provide a stable support for the tube 18, while the surface thereof to be pressed by the fingers 26 is formed from a material which has a small coefficient of friction and, hence, exhibits a small tendency of wear, e.g. a fluororesin 42B.

The operation of the peristaltic pump having the described construction is as follows:
① The tube is set in the guide recess 45 formed in the guide plate 42 and carried by the platen 23 so as to be located in the correct position in the space between the group 26A of the pressing fingers 26 and the platen 23. In this state, the pressing fingers 26 are activated successively by the operation of corresponding cams so as to be brought into their operative positions, so that the position of the collapsed portion of the tube is progressively moved in the longitudinal direction of the tube 18. Consequently, the liquid in the internal liquid passage in the tube is displaced so as to be delivered into, for example, the body of a patient, while the portion of the tube relieved from the pressing force exerted by the pressing finger 26 resumes its original form due to its elasticity so that the internal passage is expanded again to suck a new portion of the liquid, whereby a pumping action is performed.
   When one of the fingers 26 of the group 26A is actuated to exert an extraordinarily large force onto the tube 18, a correspondingly large elastic reaction force produced by the tube serves to force the movable member 22 backward away from the platen 23, against the force of the urging means 27, so that the finger 26 incorporated in this movable member 22 is moved together with the movable member 22 away from the platen 23, i. e., away from the tube 18, thereby absorbing excess portion of the collapsing force exerted by the finger 26. Consequently, the tube 18 is always held by the platen 23 in quite a stable manner and is smoothly collapsed by the successive fingers 26 under normal operating condition. In the event that an extraordinarily large pressing force is applied by one of the fingers 26, the excessive portion of the collapsing force is absorbed without fail so as to protect the tube 18 and to ensure a stable pumping operation.
   In addition, the tube 18 is received in and guided by the tube guide recess 45 formed in the guide plate 42 so that the entire portion of the tube is correctly set without winding, such that successive tube sections of a predetermined length are located in the successive areas where pressing forces are to be applied by the successive pressing fingers 26. Therefore, the tube is collapsed in quite a stable manner as the successive pressing fingers are set to operative positions, thus enhancing the precision in the control of the flow rate of the liquid under transfusion.
② The major and essential portions including the eccentric cams 25, pressing fingers 26 and the platen 23 are housed in the housing 11 by means of the door 13 so that a compact construction of the pump 10 is obtained. In addition, it is possible to set the tube 18 in the correct position between the group 26A of the pressing fingers 26 and the tube 18 very easily by the opening and closing action of the door 13.
③ The cam driving device of the pump is mounted on the movable member 22 which incorporates the eccentric cams 25, so that the actuating mechanism which operatively connects the eccentric cams 25 to the cam driving device can be set on the movable member 22, thus contributing to a simplification of the construction.
④ The urging force produced by the torsion spring 28, which backs up the movable member 22 against the elastic reaction force produced by the tube 18 is adjustable by means of the adjusting screw 29. Thus, the ability of the pressing finger 26, which is displaceable together with the movable member 22 to absorb any excessive collapsing force, is adjustable without difficulty in accordance with changes in conditions such as the elasticity of the tube 18, size of the tube 18 and so forth.
⑤ The pairs of clamping portions, which are provided at predetermined positions along the length of the guide recess 45 in the guide plate 42, effectively clamp the tube 18 so as to locate the tube 18 more precisely in the region where the collapsing pressing forces are exerted by the successive pressing fingers 26.
⑥ By pressing the clamping portions 46 on the guide plate 42 by means of the clamping fingers 47, it is possible to enhance the locating effect of the clamping portions 46 for locating the tube 18.
⑦ A compact construction of the pump 10 is obtained by virtue of the use of the housing 11 with the door 13 for accommodating the eccentric cams 25, pressing fingers 26 and the platen 23. In addition, it is possible to easily set the tube 18 in the guide plate 42 when the door is opened and closed, whereby the tube 18 can be correctly located in the space between the group 26A of the pressing fingers 26 and the platen 23.

The guide plate 42 of the pump 10 serves to stably locate the tube 18 in the region where the collapsing forces are applied by the successive pressing fingers so as to enhance the precision of control of the flow rate of the liquid, and covers the pressing window 41 in the bracket 11A so as to prevent the liquid flowing in the tube 18 from accidentally coming into the mechanical portion of the pump. Consequently, contamination of the mechanism also is prevented.

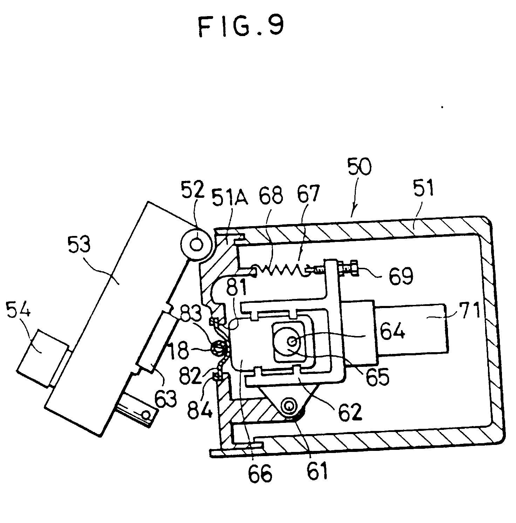

Fig. 9 shows a second embodiment of the peristaltic pump of the present invention. The peristaltic pump shown in Fig. 9 is different from the peristaltic pump of the first embodiment in that the fingers are arranged for linear movement with respect to the movable member, as will be understood from the following description.

Referring to Fig. 9, the peristaltic pump 50 of the second embodiment has, as is the case of the first embodiment, a housing 51, a door shaft 52, a door 53 and a knob 54.

The pump 50 also has a support shaft 61 which is carried at its both ends by a bracket 51A fixed to the housing 51. The support shaft 61 rockably carries a movable member 62. The pump 50 also has a platen 63 supported by the door 53.

The movable member 62 is supported for rocking motion around the support shaft 61 toward and away from the platen 63. The movable member 62 has a cam shaft 64 which extends in parallel with the support shaft 61 and incorporates a plurality of cams 65 fixedly carried by the cam shaft 64 and arranged along the length of the support shaft 61. The movable member 62 also incorporates a plurality of pressing fingers 66 which are adapted to be linearly actuated by the cams 65.

Thus, each of the pressing fingers 66 is movable between a retracted position and an operative position by the action of the associated cam 65. The platen 63 is adapted to be positioned so as to oppose the group 66A of fingers 66 so as to hold the tube 18 between itself and the pressing fingers 66. As the successive cams are operated, the associated fingers are set to the operative positions so that the position of the collapsed portion of the tube is progressively moved in the longitudinal direction of the tube.

The pump 50 further has an urging means 67 which urges the movable member 62 towards the platen 63. The urging means 67 is composed of a tensile spring 68 (urging member) and an adjusting screw 69 (adjusting member). The tensile spring 68 has one end retained by an end of the adjusting screw 69 which is screwed to the movable member 62 and the other end which is retained by the bracket 51A on the housing 51. The adjusting screw 69 can be driven into and out of the threaded hole in the bracket 51A so as to adjust the urging force produced by the tensile spring 68.

The pump 50 also has a drive motor 71 mounted on the movable member 62 and capable of driving the cam shaft 64 so as to rotate the cams 65 on the cam shaft 64, thereby operating the pressing fingers 66 corresponding to these cams 65.

As in the case of the pump 10 of the first embodiment, the pump 50 has a guide plate 82 which is disposed to cover the pressing window 81 in the bracket 51A of the housing 51 and which is provided with a tube guide recess 83 for setting the tube 18. Numeral 84 denotes a frame.

The pump 50 having the described construction operates substantially in the same manner as the pump 10 of the first embodiment.

In the first and the second embodiments as described, the movable member is supported for linear motion with respect to the housing.

As has been described, the tube is always held in a stable manner by the platen and is stably collapsed by the successive fingers under normal operating condition, whereas, in the event that an extraordinarily large force is applied, the excess portion of the collapsing force exerted by the pressing finger is effectively absorbed so as to ensure a stable pumping operation.

Furthermore, it is possible to easily adjust the level of the excess collapsing force to be absorbed, in accordance with a change in the conditions such as the elasticity of the tube, size of the tube, and so forth.

In addition, according to the invention, it is possible to stably set the tube in the right position between the group of pressing fingers and the platen.

## Claims

1. A peristaltic pump (10; 50) comprising: a plurality of eccentric cams (25, 65); a cam shaft (24, 64) carrying the cams (25, 65) and capable of driving the cams, a plurality of fingers (26, 66) adapted to be driven by the cams (25, 65) so as to press and collapse a tube; a platen (23, 63) arranged to oppose the group of fingers (26, 66) and capable of holding the tube between itself and the fingers; a tube holder (42, 82) disposed between the fingers (26) and the tube (18) and/or between the platen (23) and the tube (18); and tube holding means (46, 83) capable of holding the tube (18) in a predetermined position characterized in that the tube holding means (46, 83) is provided on the surface of the tube holder (42, 82) contactable with the tube.

2. A peristaltic pump according to claim 1, wherein the tube holder (42, 82) includes a sheet-type elastic member (42, 82) having a recess-type tube holding means formed in the surface thereof contactable with the tube (18).

3. A peristaltic pump (10) according to claim 1 or 2, wherein the tube holder includes at least a pair of tube clamping portions (46) projecting into the recess.

4. A peristaltic pump (10) according to claim 3, further comprising clamping fingers (47) capable of pressing the clamping portions (46) so as to enable the clamping portions to clamp the tube (18).

5. A peristaltic pump according to any of claims 1 to 4, comprising: a housing (11) accommodating the cams (25), a cam shaft (24) and the fingers (26) and supporting the tube holder (42, 82) in such a manner that the tube holder (42, 82) is located at a position where it opposes the tube (18) to be mounted; and a door (13) supported on the housing (11) for movement between open and closed positions and carrying the platen (23) in such a manner that the platen opposes the tube (18).

6. A peristaltic pump according to any of claims 1 to 5, wherein the surface of the tube holder (42) to be contacted by the tube (18) is made of a silicon rubber (42A) having a large coefficient of friction, while the surface of the tube holder (42) to be contacted by the fingers (26) is made of a fluororesin (42B) having a small coefficient of friction.

## Patentansprüche

1. Peristaltische Pumpe (10; 50), umfassend: eine Anzahl von Exzenternocken (25, 65); eine Nockenwelle (24, 64), die die Nocken (25, 65) trägt und in der Lage ist, die Nocken anzutreiben, eine Anzahl von Fingern (26, 66), die durch die Nocken (25, 65) antreibbar sind, um einen Schlauch druckzubeaufschlagen und zusammenzudrücken; eine Platte (23, 63), die angeordnet ist, so daß sie der Gruppe der Finger (26, 66) gegenüberliegt und in der Lage ist, den Schlauch zwischen sich und den Fingern zu halten; einen Schlauchhalter (42, 82), der zwischen den Fingern (26) und dem Schlauch (18) und/oder zwischen der Platte (23) und dem Schlauch (18) angeordnet ist; und ein Schlauchhaltemittel (46, 83), das in der Lage ist, den Schlauch (18) in einer vorbestimmten Position zu haltern, dadurch **gekennzeichnet,** daß das Schlauchhaltemittel (46, 83) auf der Oberfläche des Schlauchhalters (42, 82) vorgesehen ist, die mit dem Schlauch in Kontakt bringbar ist.

2. Peristaltische Pumpe nach Anspruch 1, bei der der Schlauchhalter (42, 82) ein elastisches Element (42, 82) vom Typ einer dünnen Platte umfaßt, das ein in seiner mit dem Schlauch (18) in Kontakt bringbaren Außenfläche gebildetes Schlauchhaltemittel vom Typ mit Vertiefung aufweist.

3. Peristaltische Pumpe (10) nach Anspruch 1 oder 2, bei der der Schlauchhalter wenigstens ein Paar von Schlauchklemmabschnitten (46) umfaßt, die in die Vertiefung vorstehen.

4. Peristaltische Pumpe (10) nach Anspruch 3, weiter umfassend Klemmfinger (47), die in der Lage sind, die Klemmabschnitte (46) derart druckzubeaufschlagen, daß die Klemmabschnitte den Schlauch (18) klemmen können.

5. Peristaltische Pumpe nach einem der Ansprüche 1 bis 4, umfassend: ein Gehäuse (11), in dem die Nocken (25), eine Nockenwelle (24) und die Finger (26) untergebracht sind, und das den Schlauchhalter (42, 82) auf solche Weise abstützt, daß sich der Schlauchhalter (42, 82) an einer Position befindet, wo er dem anzubringenden Schlauch gegenüberliegt; und eine am Gehäuse (11) gehalterte Tür (13) zur Bewegung zwischen einer offenen und einer geschlossenen Position, die die Platte (23) auf solche Weise trägt, daß die Platte dem Schlauch (18) gegenüberliegt.

6. Peristaltische Pumpe nach einem der Ansprüche 1 bis 5, bei der die durch den Schlauch (18) zu kontaktierende Fläche des Schlauchhalters (42) aus Silikongummi (42A) hergestellt ist, das einen großen Reibungskoeffizienten aufweist, während die durch die Finger (26) zu kontaktierende Fläche des Schlauchhalters (42) aus einem Fluorharz (42B) mit einem kleinen Reibungskoeffizienten hergestellt ist.

## Revendications

1. Pompe péristaltique (10 ; 50) comprenant : une pluralité de cames excentriques (25 ; 65), un arbre à cames (24 ; 64) qui porte lesdites cames (25 ; 65) et est capable d'entraîner lesdites cames, une pluralité de doigts (26 ; 66) aptes à être entraînés par lesdites cames (25 ; 65) de façon à comprimer et écraser un tube, un plateau (23 ; 63) disposé pour être en vis-à-vis dudit groupe de doigts (26 ; 66) et capable de maintenir le tube entre lui-même et les doigts, un support de tube (42 ; 82) disposé entre les doigts (26) et le tube (18) et/ou entre le plateau (23) et le tube (18), et un moyen (46 ; 83) de maintien de tube capable de maintenir le tube (18) dans une position prédéterminée, caractérisée en ce que le moyen (46 ; 83) de maintien de tube est placé sur la surface du support de tube (42 ; 82) avec laquelle le tube peut venir en contact.

2. Pompe péristaltique selon la revendication 1, dans laquelle le support de tube (42 ; 82) comprend un élément élastique (42 ; 82) en forme de feuille, dans la surface pouvant venir en contact avec le tube (18) duquel est formé un moyen de maintien de tube en forme de cavité.

3. Pompe péristaltique (10) selon la revendication 1 ou 2, dans laquelle le support de tube comprend au moins une paire de parties (46) de serrage de tube qui dépassent dans la cavité.

4. Pompe péristaltique (10) selon la revendication 3, comprenant en outre des doigts de serrage (47) capables d'appuyer sur les parties de serrage (46) de manière à permettre aux parties de serrage de serrer le tube (18).

5. Pompe péristaltique selon l'une quelconque des revendications 1 à 4, comprenant : une enceinte (11) abritant les cames (25), un arbre à cames (24) et les doigts (26) et supportant le support de tube (42 ; 82) de manière telle que le support de tube (42 ; 82) est situé en une position où il est en face du tube (18) à monter, et une porte (13) montée sur ladite enceinte (11) en vue d'un déplacement entre des positions ouverte et fermée et supportant le plateau (23) de telle manière que le plateau est en face du tube (18).

6. Pompe péristaltique selon l'une quelconque des revendications 1 à 5, dans laquelle la surface du support de tube (42) avec laquelle doit venir en contact le tube (18) est faite d'un caoutchouc au silicone (42A) ayant un fort coefficient de frottement, tandis que la surface du support de tube (42) destinée à venir en contact avec les doigts (26) est faite d'une résine fluorée (42B) ayant un faible coefficient de frottement.
